# EUROPÄISCHE PATENTSCHRIFT

(11) **EP 0 989 909 B1**
(45) Veröffentlichungstag und Bekanntmachung des Hinweises auf die Patenterteilung: **19.09.2001**
(21) Anmeldenummer: 98930783.0
(22) Anmeldetag: 02.06.1998
(51) Int. Cl.: B01J 31/16, B01J 31/22, C07C 67/00

(54) **AUS PHOSPHINOALKYL-FUNKTIONALISIERTEM POLYSTYROL ERHÄLTLICHER KATALYSATOR UND VERFAHREN ZUR HERSTELLUNG VON DELTA-LACTON**
CATALYST OBTAINED FROM PHOSPHINOALKYL-FUNCTIONALIZED POLYSTYRENE AND METHOD FOR THE PRODUCTION OF DELTA-LACTONE
CATALYSEUR S'OBTENANT A PARTIR DE POLYSTYRENE A VALENCE FONCTIONNELLE PHOSPHINOALKYLE ET PROCEDE POUR LA PREPARATION DE DELTA-LACTONE

(30) Priorität: 18.06.1997 DE 19725735
(43) Veröffentlichungstag der Anmeldung: 05.04.2000
(73) Patentinhaber: Forschungszentrum Karlsruhe GmbH, 76133 Karlsruhe (DE)
(72) Erfinder: PITTER, Stephan, D-07743 Jena (DE); DINJUS, Eckhard, D-07743 Jena (DE); HOLZHEY, Nancy, D-07768 Kahla (DE)
(86) Internationale Anmeldenummer: EP9803290
(87) Internationale Veröffentlichungsnummer: WO9857745

(56) Entgegenhaltungen:
- WO-A-95/15815
- US-A- 4 506 030

## Beschreibung

Die Erfindung betrifft einen aus phosphinoalkyl-funktionalisiertem Polystyrol erhältlichen Katalysator gemäß dem ersten Patentanspruch sowie ein Verfahren zur Herstellung von δ-Lacton gemäß dem Oberbegriff des dritten Patentanspruchs.

Aus K. Kaneda et al. "Selective Telomerization of Butadiene with Various Nucleophiles Catalyzed by Polymer-Bound and Palladium(0) Complexes", J. Org. Chem. 1981, 46, 2356 - 2362 ist die Verwendung von phosphinoalkly-funktionalisiertem Polystyrol zur Herstellung eines Katalysators bekannt, der Umsetzungen von 1,3-Butadien mit Nucleophilen katalysiert.

Aus der Veröffentlichung von Stephan Pitter, Eckhard Dinjus, Beate Jung und Helmar Görls: "Phosphinoalkylnitrile: Synthese und Koordinationsverhalten an Palladiumzentren", Z. Naturforsch. **51 b**, 934-946 (1996) ist es bekannt, daß die Synthese des δ-Lactons (2-Ethyliden-hepta-6-en-5-olid) mit hoher Selektivität durch Pd(0)-Komplexe mit sperrigen Phosphinliganden katalysiert wird. Als Vorstufe zur Darstellung der Katalysatoren wird ein Phosphinoalkylnitril des Typs R₂P-(CH₂)ₙ-CN (n = 1) eingesetzt, wobei Kettenlängen mit n > 4 aus sterischen Gründen bevorzugt werden. Das Phosphinoalkylnitril wird mit einer Palladium(II)-Verbindung, z. B. (η⁵-cyclopentadienyl) (η³-allyl)palladium, umgesetzt. Die Synthese des δ-Lacton erfolgt damit durch homogene Katalyse unter Verwendung eines Lösungsmittels.

Die EP 0 234 668 A2 beschreibt die Herstellung des δ-Lactons durch Umsetzung von 1,3-Butadien mit Kohlendioxid in einem Lösungsmittel in Gegenwart von Palladium oder einer Palladiumverbindung, eines Amins und eines zweizähnigen Liganden der allgemeinen Formel R¹R²MRMR³R⁴, wobei M beispielsweise Phosphor, R eine organische überbrückende Gruppe mit 3 C-Atomen und R¹⁻⁴ substituierte oder unsubstituierte Kohlenwasserstoffgruppen sind.

Auch aus der DE 28 38 610 A geht ein Verfahren zur Herstellung von δ-Lacton aus 1,3-Butadien und Kohlendioxid hervor. Bei diesem Verfahren erfolgt die Umsetzung der beiden Ausgangsstoffe in Gegenwart eines Katalysators in Form eines Palladiumphosphin-Komplexes der Formel Pd[P(R)₃]ₓ, worin x eine ganze Zahl von 2 bis 4 und R einen Alkylrest oder Cycloalkylrest mit bis zu 8 Kohlenstoffatomen oder einen Phenylrest darstellt. Die Reste können gleichfalls substituiert und gleich oder verschieden sein. Als Verfahrenstemperatur sind 20° bis 150° C und als Gesamtdruck 10 bis 500 bar angegeben. Der Katalysator läßt sich in situ herstellen, indem dessen Edukte, eine Palladiumverbindung und ein Phosphin, dem Reaktionsmedium zugegeben werden.

Die bekannten Verfahren mit homogener Katalyse weisen einige Nachteile auf. Der Katalysator läßt sich nicht wieder zurückgewinnen, so daß die teuren Ausgangsstoffe für jede Umsetzung erneut zur Verfügung stehen müssen. Außerdem verunreinigen der Katalysator und seine Abbauprodukte das gewünschte Endprodukt.

Aufgabe der Erfindung ist es, diesen Nachteilen abzuhelfen und bei der Umsetzung von Butadien mit Kohlendioxid einen solchen Katalysator einzusetzen, der sich einfach zurückgewinnen läßt und das Endprodukt nicht verunreinigt. Außerdem soll ein weiteres Verfahren zur Herstellung von δ-Lacton vorgeschlagen werden.

Die Lösung der Aufgabe ist im Patentanspruch 1 sowie im Kennzeichen von Patentanspruch 3 beschrieben. Patentanspruch 2 gibt eine bevorzugte Ausgestaltung des Katalysators an.

Erfindungsgemäß wird zur Herstellung des Katalysators ein beliebiges phosphinoalkyl-funktionalisiertes Polystyrol eingesetzt, wobei es von untergeordneter Bedeutung ist, in welchen Abständen die am Polymergerüst hängenden Phenylgruppen substituiert sind. Die Phosphinogruppe kann mit einem geradkettigen oder verzweigten Alkyl-, beispielsweise mit 2 bis 8 Kohlenstoffatomen, mit einem Cycloalkyl- oder mit einem Arylrest sub stituiert sein. Bevorzugt wird die Substituierung mit einem Isopropylrest.

Das phosphinoalkyl-funktionalisierte Polystyrol (2) läßt sich beispielsweise durch die folgende Reaktion aus kommerziell erhältlichem, partiell chlormethyliertem Polystyrol (1) herstellen: 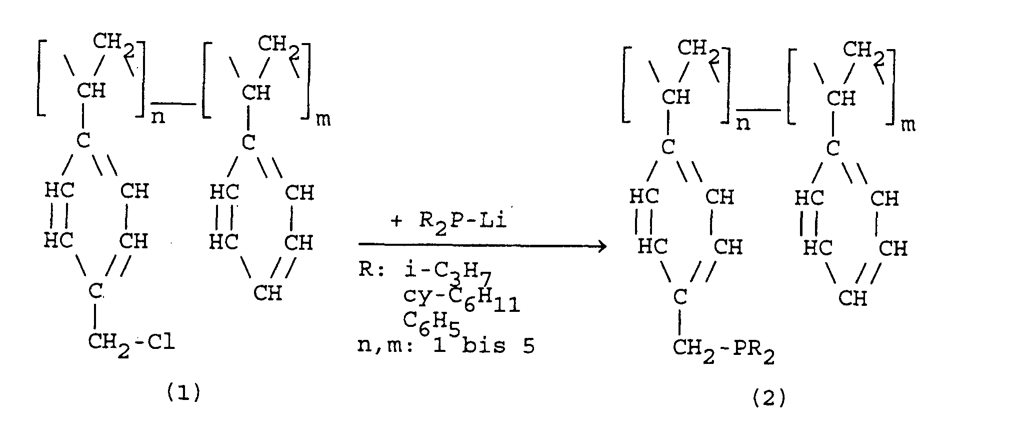 wo R ein Alkyl, Aryl oder Cycloalkyl, vorzugsweise Isopropyl, ist und n und m gleiche oder verschiedene ganze Zahlen von 1 bis 5 darstellen.

Zur Herstellung des Katalysators wird das phosphinoalkyl-funktionalisierte Polystyrol mit (η⁵-cyclopentadienyl) (η³-allyl)palladium (3) umgesetzt.

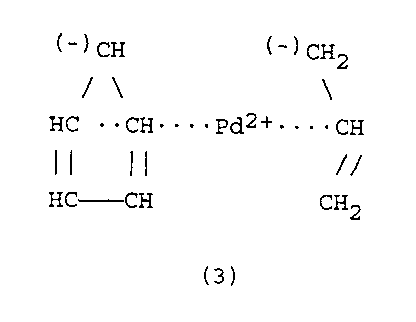

Das Allyl kann substituiert sein, etwa mit Alkylketten von 1 bis 10 C-Atomen Länge. In gleicher Weise kann der Cyclopentadienylrest substituiert sein. Bevorzugt wird jedoch unsubstituiertes Allyl (C₃H₅⁻). Die Umsetzung kann "in situ" in Gegenwart der Reaktanden oder aber vor der Reaktion erfolgen. Die zweite Alternative eröffnet die beim Stand der Technik nicht gegebene Möglichkeit, vorpräparierten Katalysator einzusetzen.

Das Befüllen des Reaktors erfolgt unter Schutzgasatmosphäre (Stickstoff, Argon). Die Reaktion wird unter Ausschluß von Luft und Feuchtigkeit unter einer Kohlendioxid-Atmosphäre bei einer Temperatur zwischen 25° und 90°C, bevorzugt bei 60° bis 70° C und einem Druck von 5 bis 100 bar, bevorzugt bei 20 bis 30 bar, durchgeführt. Die Reaktionsdauer beträgt 2 bis 50 h.

Sowohl der "in situ" als auch der vorpräparierte Katalysator lassen sich mehrfach wiederverwenden. Die Aktivität des vorpräparierten Katalysators ist anfangs höher, sinkt jedoch nach einigen Zyklen auf mit dem in situ erzeugten Katalysator vergleichbare Werte. Die Selektivität in der Bildung von 2-(E)-Ethyliden-hepta-6-en-5-olid liegt bei nicht zu langen Reaktionszeiten zwischen 57 und 71 %.

Die nach Pitter et al und der dort zitierten Literatur alternativ zugänglichen Produkte (C₁₇-Ester, γ-Lacton etc.) lassen sich ebenfalls mit dem hier beschriebenen Katalysatorsystem herstellen (z. B. bei deutliche verlängerten Reaktionszeiten).

Die Erfindung bietet eine Reihe von Vorteilen: Der Katalysator läßt sich leicht durch Filtration vom Reaktionsgemisch abtrennen und und durch einfaches Waschen und Trocknen regenerieren. Alternativ kann der Katalysator in einem Sieb vorgelegt werden. In Bezug auf den Einsatz des teuren Butadiens zeigt er eine hohe Selektivität, die im Bereich zwischen 50 und 75 % liegt, so daß die Butadienverluste durch Umsetzung zu Nebenprodukten gering sind. Das Endprodukt ist nicht mit dem Katalysator oder seinen Abbauprodukten verunreinigt. Das Verfahren läßt sich somit auch kontinuierlich durchführen.

Die Erfindung wird im folgenden anhand von Versuchsbeispielen näher erläutert.

In allen Fällen wurde als Katalysator (η⁵-cyclopentadienyl) (η³-allyl)palladium eingesetzt. Als Lösungsmittel wurde Acetonitril verwendet. Die Reaktionsbedingungen waren 16 h bei ca. 70° C.

Alle Versuche wurden in einem 250 ml Edelstahlautoklaven, ausgestattet mit Gaseinlaß- und auslaßventilen, Manometer, Innenthermometer, Magnet-Rührstäbchen und beheizbarem Magnetrührer, durchgeführt. Die angegebenen Reaktionstemperaturen wurden mittels eines Ölbades eingestellt. Um die Einzelversuche innerhalb einer Reihe in bezug auf die Selektivität zum δ-Lacton vergleichen zu können, wurden die Reaktionszeiten so gewählt, daß noch kein vollständiger Umsatz von Butadien erfolgt ist.

### 1. Beispiel

### Katalysen mit in situ hergestelltem Katalysator

227,6 mg [(Diisopropylphosphino)methyl]polystyrol (mit 0,333 mmol gebundenem Phosphor) werden in 20 ml Acetonitril aufgerührt und mittels einer PTFE-Kanüle in den Autoklaven überführt. Anschließend werden 36,8 mg (η⁵-cyclopentadienyl) (η³-allyl)palladium (0,173 mmol) gelöst in 10 ml Acetonitril ebenfalls über eine PTFE-Kanüle zugegeben. Nachdem der Autoklav auf -30° C abgekühlt worden ist, werden 13,5 g 1,3-Butadien (0,25 mmol) über eine Edelstahlleitung einkondensiert. Der Autoklav wird auf 25° C erwärmt. Dann werden 13,2 g CO₂ (0,3 mol) aufgepreßt. Nun wird der Autoklav auf 70° C temperiert, wobei der Innendruck auf 25 bis 27 bar ansteigt. Es wird mit 500 Umdrehungen/min gerührt. Nach 16 h Reaktionsdauer wird der Reaktor außerhalb des Ölbades auf 25° C abgekühlt und der Überdruck vorsichtig innerhalb von 30 min über eine Abgasleitung abgelassen. Die in der Regel gelbe Reaktionslösung wird durch Filtration über eine G4-Fritte vom polymeren Katalysator getrennt. Nach Einengen der Reaktionslösung bei 40° C im Ölpumpenvakuum wird ausgewogen und die Ausbeute an Ù-Lacton mittels HPLC bestimmt. Der abgetrennte Katalysator wird noch zweimal mit je 10 ml Acetonitril gewaschen, in ein Schlenkgefäß überführt und bei 25° C im Vakuum von Lösungsmittelresten befreit. Er kann nun für weitere Katalysen verwendet werden.

Alle weiteren Katalysen können analog der oben beschriebenen Vorgehensweise durchgeführt werden, mit dem Unterschied, daß der getrocknete heterogene Katalysator aus vorherigen Versuchen in 30 ml Acetonitril aufgenommen und mittels PTFE-Kanüle in den Autoklaven überführt wird. Die beiden nachfolgend aufgeführten Versuchsreihen wurden unter identischen Bedingungen durchgeführt und demonstrieren die gute Reproduzierbarkeit des Verfahrens.

| 1. Versuchsreihe | Masse des Rohprodukts in [g] | Selektivität in [%] |
|---|---|---|
| 1. Einsatz des Kat.: | 2,413 | 57 |
| 2. Einsatz des Kat.: | 1,326 | 64 |
| 3. Einsatz des Kat.: | 1,126 | 71 |
| 4. Einsatz des Kat.: | 1,117 | 66 |
| 5. Einsatz des Kat.: | 0,938 | 63 |

| 2. Versuchsreihe | Masse des Rohprodukts in [g] | Selektivität in [%] |
|---|---|---|
| 1. Einsatz des Kat.: | 2,513 | 60 |
| 2. Einsatz des Kat.: | 1,459 | 65 |
| 3. Einsatz des Kat.: | 1,011 | 61 |
| 4. Einsatz des Kat.: | 1,100 | 67 |
| 5. Einsatz des Kat.: | 0,976 | 64 |

| Langzeitversuch: | | |
|---|---|---|
| 232,5 h bei 73 - 75°C | Masse des Rohprodukts in [g] | Selektivität in [%] |
| | 9,520 | 38 |

### 2. Beispiel:

### Katalysen mit vorpräpariertem Katalysator

Zur Vorpräparation eines Katalysators aus [(Diisopropylphosphino)methyl]polystyrol und (η⁵-cyclopentadienyl) (η³-allyl)palladium werden zu 227,6 mg [(Diisopropylphosphino)methyl]polystyrol (mit 0,333 mmol gebundenem Phosphor) in 20 ml Acetonitril unter kräfigem Rühren bei 25° C 36,8 mg (η⁵-cyclopentadienyl) (η³-allyl)palladium (0,173 mmol) gelöst in 10 ml Acetonitril zugegeben. Die Reaktionsmischung wird vier Stunden bei 60° C gehalten. Anschließend wird auf 25° C abgekühlt, der Katalysator abfiltriert und fünfmal mit jeweils 25 ml Acetonitril gewaschen. Nach Trocknung im Ölpumpenvakuum bei 25° C wird ein dunkelbrauner Feststoff erhalten.

Der in 30 ml Acetonitril aufgerührte Katalysator wird mittels PTFE-Kanüle in den Autoklaven überführt. Die weitere Durchführung der Reaktion erfolgt analog zu Beispiel 1.

| | Masse des Rohprodukts in [g] | Selektivität in [%] |
|---|---|---|
| 1. Einsatz des Kat.: | 4,692 | 56 |
| 2. Einsatz des Kat.: | 1,953 | 68 |
| 3. Einsatz des Kat.: | 1,376 | 72 |
| 4. Einsatz des Kat.: | 1,211 | 71 |
| 5. Einsatz des Kat.: | 0,949 | 66 |

| Langzeitversuch: | | |
|---|---|---|
| 232 h bei 73 - 75°C | Masse des Rohprodukts in [g] | Selektivität in [%] |
| | 9,558 | 37 |

## Patentansprüche

1. Katalysator, erhältlich durch Umsetzung von phosphinoalkylfunktionalisiertem Polystyrol mit (η⁵-cyclopentadienyl) (η³-allyl)palladium.

2. Katalysator nach Anspruch 1, erhältlich durch Umsetzung mit (Diisopropylphosphino)methyl-funktionalisiertem Polystyrol.

3. Verfahren zur Herstellung von δ-Lacton, bei dem man 1,3-Butadien mit Kohlendioxid in Anwesenheit eines Katalysators umsetzt, wobei als Katalysator das Produkt der Reaktion von (η⁵-cyclopentadienyl)(η³-allyl)palladium mit einer Verbindung, die eine Phosphinogruppe enthält, eingesetzt wird, **dadurch gekennzeichnet, daß**
die Verbindung, die eine Phosphinogruppe enthält, ein phosphinoalkyl-funktionalisiertes Polystyrol ist.

## Claims

1. Catalyst, obtainable by reacting phosphinoalkyl-functionalised polystyrene with (η⁵-cyclopentadienyl) (η³-allyl)palladium.

2. Catalyst according to claim 1, obtainable by reacting with (diisopropylphophino)methyl-functionalised polystyrene.

3. Method of producing δ-lactone, wherein 1,3-butadiene is reacted with carbon dioxide in the presence of a catalyst, the product of the reaction of (η⁵-cyclopentadienyl) (η³-allyl)palladium with a compound, which contains a phosphino group, being used, **characterised in that** the compound, which contains a phosphino group, is a phosphinoalkyl-functionalised polystyrene.

## Revendications

1. Catalyseur accessible par réaction de polystyrène fonctionnalisé par un phosphinoalkyle avec le (η⁵-cyclopentadiényl) (η³-allyl)palladium.

2. Catalyseur selon la revendication 1, accessible par réaction avec un polystyrène fonctionnalisé par du (diisopropylphosphino)méthyle.

3. Procédé de préparation de δ-lactone dans lequel on fait réagir le 1,3-butadiène avec du dioxyde de carbone en présence d'un catalyseur, procédé dans lequel on met en oeuvre comme catalyseur le produit de la réaction du (η⁵-cyclopentadiényl) (η³-allyl)palladium avec un composé qui contient un groupe phosphine,
**caractérisé en ce que**
le composé qui contient un groupe phosphine est un polystyrène fonctionnalisé par un groupe phosphinoalkyle.
